# EUROPEAN PATENT APPLICATION

(11) **EP 4 696 217 A1**
(43) Date of publication of application: **18.02.2026**
(21) Application number: 24194903.1
(22) Date of filing: 16.08.2024
(51) Int. Cl.: A61B 3/00, A61B 3/14, A61B 3/10

(54) **MULTI-MODAL OPHTHALMIC IMAGING INSTRUMENT OPERABLE IN A PLURALITY OF IMAGING MODALITIES**

(71) Applicant: Optos plc, Dunfermline, Scotland KY11 8GR (GB)
(72) Inventor: THOMSON, Martin, Dunfermline, Scotland, KY11 8GR (GB); GEDDES, David, Dunfermline, Scotland, KY11 8GR (GB)
(74) Representative: Hoffmann Eitle

(57) **Abstract**

An ophthalmic imaging instrument comprising a plurality of light sources for providing a beam of light; a controller arranged to control the light sources to generate a beam of light and to provide a plurality of imaging modalities, wherein a corresponding contribution from the light sources to a power of light projected toward an eye of a subject varies for each one of said imaging modalities; an optical path comprising one or more scanning elements and one or more guiding elements configured to project the beam of light to an eye of the subject; and a shutter arrangement configured to block the beam of light so that it does not reach the eye of the subject, wherein the controller is configured to operate the shutter arrangement to block the beam of light when switching between a first imaging modality and a second imaging modality.

## Description

### Technical field

The present invention relates to a multi-modal ophthalmic imaging instrument operable in a plurality of imaging modalities, for which a contribution of a corresponding light source to a beam of light varies. More particularly but not exclusively, the present invention relates to switching between a first imaging modality and a second imaging modality for which the contribution of the light sources to a beam of light toward the eye of a subject varies.

### Background

Ophthalmic imaging instruments are widely used to image a patient's eye in order to assess the health of the eye. Such systems comprise one or more light sources and a controller that is arranged to control the light sources and the instrument to generate a beam of light that is to scan the tissue of interest. An optical path in such instruments comprises one or more scanning elements and one or more guiding elements configured to project the beam of light to an eye of the subject. For example, one or more polygon scanning mirrors and one or more galvanometer scanning mirrors implement the beam scanning. Further, a beam splitter is usually provided for splitting a beam of light into two, wherein one partial beam propagates towards the eye of a subject and the other partial beam is directed at a detector which translates the measured power into some signal that is indicative of the optical power incident into the detector. Assuming a well-defined and constant splitting ratio of the beam splitter, the optical power that is actually directed toward an eye can be controlled by means of controlling the output power of the light source on the basis of the signal being indicative of the optical power of the partial beam that is incident into the detector.

As in ophthalmic imaging the imaging target is a subject's eye, appropriate power control may be an important aspect. On the one hand, the optical power of light incident into the eye must not exceed a given power budget at any time, as otherwise the light may damage tissue. On the other hand, however, the optical power needs to be high enough in order to obtain a sufficient imaging quality. Notably, the signal to noise ratio may become undesirably low if the optical power of the incident beam is too weak. Therefore, common ophthalmic imaging instruments comprise control mechanisms that keep the optical power inside a permissible power band, i.e. below a maximum threshold power in order to prevent damage to a subject's eye, and above a minimum threshold in order to provide the desired imaging quality. Such control mechanisms usually shut the light source down or prevent the light from reaching a subject's eye by other means whenever the light beam power moves outside the permissible band.

Further, modern ophthalmic imaging instruments can operate in multiple imaging modalities such that the clinician can select an imaging modality that is most suited to imaging a given region or disease in the patient's eye. Each imaging modality allows the ophthalmic imaging system to acquire a different type of ophthalmic image, which helps the clinician to acquire images containing information most appropriate for diagnosing ocular disease. For example, a colour imaging modality may be used to acquire a fundus image of a patient's ocular fundus, including a portion of the retina. In this imaging modality, a combination of red, green and blue laser light may be delivered to the patient's eye to acquire images of the ocular fundus. Infrared (IR) retinal imaging and auto-fluorescence (AF) imaging are other examples of imaging modalities that may be used to image a patient's eye. The former IR imaging delivers light from an infrared laser to the patient's eye, and may be used to detect retinal pathologies such as intraretinal fluid or a retinal pigment epithelium tear. The latter AF imaging employs usually a monochromatic light beam of only one laser which excites fluorescent matter for imaging. The respective wavelengths of the incident light and the fluorescent response are different, so that the actual tissue response can be distinguished from the illumination.

As power control remains essential even under varying imaging conditions or modalities, the existing power control mechanisms oftentimes fail to accommodate for both sufficient and reliable control of light beam powers as well as the desired flexibility. There is therefore a need for improved power control mechanisms in ophthalmic imaging instruments that allow for the flexibility but maintain safety while keeping system complexity at an acceptable level.

### Summary

Problems are solved and objects are met by the subject matter of the independent claims. Further preferred embodiments are defined in the dependent claims.

According to one embodiment of the present invention, there is provided an ophthalmic imaging instrument comprising a plurality of light sources for providing a beam of light; a controller arranged to control the light sources to generate a beam of light and to provide a plurality of imaging modalities, wherein a corresponding contribution from the light sources to a power of light projected toward an eye of a subject varies for each one of said imaging modalities; an optical path comprising one or more scanning elements and one or more guiding elements configured to project the beam of light to an eye of the subject; and a shutter arrangement configured to block the beam of light so that it does not reach the eye of the subject, wherein the controller is configured to operate the shutter arrangement to block the beam of light when switching between a first imaging modality and a second imaging modality.

According to a method embodiment of the present invention there is provided a method of operating an ophthalmic imaging instrument comprising a plurality of light sources for providing a beam of light and an optical path comprising one or more scanning elements and one or more guiding elements configured to project the beam of light to an eye of the subject, and a shutter arrangement configured to block the beam of light so that it does not reach the eye of the subject, the method comprising the steps of controlling the light sources to generate a beam of light and to provide a plurality of imaging modalities, wherein a corresponding contribution from the light sources to a power of light projected toward an eye of a subject varies for each one of said imaging modalities; and operating the shutter arrangement to block the beam of light when switching between a first imaging modality and a second imaging modality.

### Brief Description of the Drawings

Embodiments of the present invention, which are presented for better understanding of the inventive concepts, but which are not to be seen as limiting the invention, will now be described with reference to the figures in which:
- Figure 1: shows a schematic view of an ophthalmic imaging instrument according to a general device embodiment of the present invention;
- Figures 2A & 2B: show schematic views of implementations of a shutting arrangement in ophthalmic imaging instruments according to more specific device embodiments of the present invention;
- Figure 3: shows a schematic view of an ophthalmic imaging instrument for providing more than one imaging modality according to a device embodiment of the present invention;
- Figure 4: shows schematic views of optical powers for individual beams of light under different imaging modalities and other related driving signals according to an embodiment of the present invention;
and
- Figure 5: shows a flowchart of a general method embodiment of the present invention.

### Detailed Description

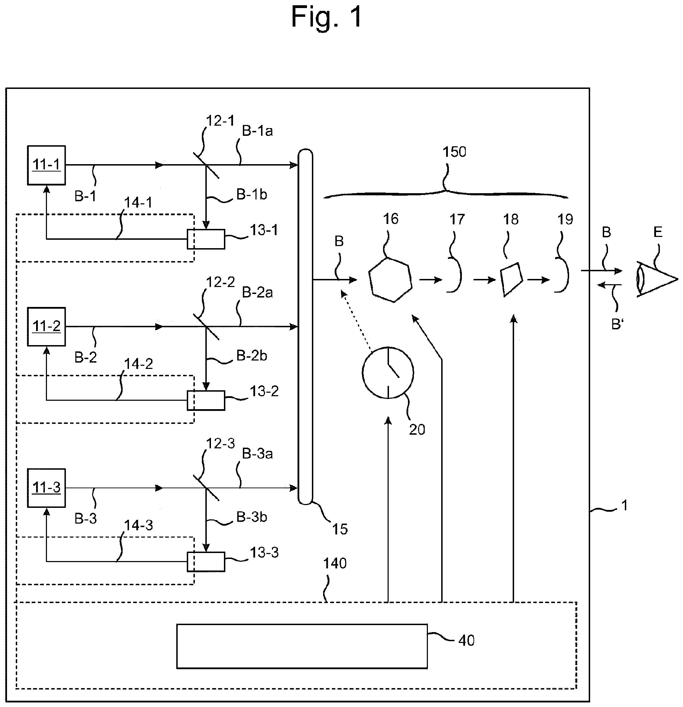

Figure 1 shows a schematic view of an ophthalmic imaging instrument according to a general device embodiment of the present invention. The shown ophthalmic imaging instrument is intended for imaging a subject's (patient's) eye in order to - for example - assess the health of the eye. The present embodiment relates, in particular but not exclusively, to controlling the optical power of one or more light sources that contribute to a beam of light used for imaging an eye of a subject. The shown ophthalmic imaging instrument 1 comprises a plurality of light sources 11-1, 11-2, ... each providing a respective beam of light B-1, B-2,... . For example, the ophthalmic imaging instrument 1 may comprise the plurality of light sources in the form of laser light sources such as a red (R) laser, a green (G) laser and a blue (B) laser. Other type light sources include one or more infrared (IR) laser(s) and one or more ultraviolet (UV) laser(s). The individual beams of light B-1, B-2, ... may be combined in a combining optics 15 which is configured to combine, e.g. collimate, individual beams to form one single beam with the respective colour components following the respective individual contributions from the individual light sources. In this way, the plurality of light sources are configured to provide a beam of light in the form of the scanning beam B which is directed toward an eye E of a subject.

The ophthalmic imaging instrument 1 may, as in the present example embodiment, be provided in form of an ultra-widefield (UWF) ophthalmic imaging instrument, which has a larger field of view (FoV) than a WF (widefield) instrument and is operable to acquire an UWF image of the ocular fundus covering a larger proportion of the ocular fundus. An UWF image of the ocular fundus is defined as a single-capture image, centred on the fovea of the eye E, which captures retinal anatomic features in the far periphery that are anterior to the vortex vein ampulla in all four quadrants. An UWF ophthalmic imaging instrument can thus acquire an UWF image being a single-capture image which covers a region of the retina extending from the fovea to the anterior edge of vortex vein ampulla and beyond to pars plana, and is defined to have a FoV (expressed in terms of the eye-angle) between 110 degrees and 220 degrees. By way of an example, the Optos Daytona^{™} is capable of UWF images (so -called Optomap^{™} images) covering up to 200 degrees of the fundus (or approximately 82% of the retina) in a single capture.

It should be noted that the techniques described herein are not limited in their applicability to WF and UWF ophthalmic imaging instruments but are also applicable to ophthalmic imaging instruments having a smaller FoV, and may benefit any scanning ophthalmic imaging instrument which scans light across a portion of the eye E via one or more ellipsoidal mirrors to image the portion.

The shown ophthalmic imaging instrument 1 comprises a controller 140 arranged to control the light sources 11-1, 11-2,... to generate the beam of light B and to provide a plurality of imaging modalities, wherein a corresponding contribution from the light sources 11-1, 11-2, ... to a power of light projected toward an eye E of the subject varies for each one of said imaging modalities. As part of this power control, there may be provided a configuration of one or more respective beam splitters 12-1, 12-2,... and power detectors 13-1, 13-2,... In such a setup, one partial beam B-1a propagates towards the eye of the subject and the other partial beam B-1b is directed at a detector, e.g. detector 13-1, which translates the measured power into some signal that is indicative of the optical power incident into the detector. Assuming a well-defined and constant splitting ratio of the beam splitter, the optical power that is actually directed toward an eye can be controlled by means of respective feedback control loops 14-1, 14-2, .... These individual power controls can be employed to provide respective target optical powers for the light sources 11-1, 11-2, ..., in turn providing a plurality of imaging modalities.

For example, the controller can be arranged to control a first number of light sources to provide a first imaging modality, and to control a second number, being different from said first number, of light sources to provide a second imaging modality. In such an example with a plurality of light sources, respective lasers can emit light in a red colour (R), a green colour (G) and in a blue colour (B), and a first imaging modality may be provided by having only one light source being active and using the available optical budget for a permissible power. This imaging modality can be a green auto-fluorescence (G-AF) imaging modality, in which only green light is used. In a second imaging modality all three light sources R, G and B can be active and each light source can only use a fraction of the optical power budget. For example, each light source can be operated to provide only a third of the optical power so as to provide a full colour or RGB imaging modality, in which white light is used.

The ophthalmic imaging instrument 1 further comprises an optical path 150 that is configured to project the beam of light B to an eye E of the subject. The optical path 150 comprises one or more scanning elements. Thus, the beam of light B can be deflected by one or more scan relay elements so as to cover an imaging area by means of a parallel arrangement of a plurality of scanning lines. The reflecting or scan relay element(s) can comprise a polygon scanning mirror 16 and one or more scanning galvanometer mirrors 18 (hereafter referred to as a "galvo" 18). The former polygon scanning mirror 16 can comprise a plurality of reflecting facets that are arranged along a circumference of a rotating body. A driver is arranged to rotate during operation the body of the polygon scanning mirror, such that each facet can reflect an incident beam of light at a varying angle. During the passing of one individual facet, the beam of light is deflected in a corresponding range of a varying angle. After completion of such a deflection cycle, the next facet substantially repeats this process and so do all other facets.

This repeated deflection by such a polygon scanning mirror 16 can form the basis for at least one scanning direction by means of providing a plurality of parallel scanning lines. A further scanning element, such as an already mentioned galvo 18, can then further deflect the beam in another direction so as to displace the scanning lines from each other on the target and to ultimately cover a two-dimensional scanning area. At least in some imaging modalities, the light from the scanning beam is scattered back from the target tissue which includes in the case of ophthalmic imaging primarily parts of the human eye, such as the retina. In general, the light B' thus travels back along the incident light path so as to be ultimately detected by an imaging detector in the form of a light sensor, converted into an intensity signal, and processed to form the image. Especially the latter signal processing is implemented by means of digital processing resources that compile individual images from corresponding sets of line scans. The optical path 150 comprises further one or more guiding elements, in the form of, for example, ellipsoidal mirrors 17, 19. Other examples for guiding elements include lenses, mirrors, waveguides, and the like. Generally, the components and elements of the optical path 150 are configured to guide the beam of light B to an eye E of a subject, scan an imaging area of the target tissue, and to guide the beam of light B' back from the eye E to be detected.

The ophthalmic imaging instrument 1 further comprises a shutter arrangement 20 that is configured to block the beam of light B so that it does not reach the eye E of the subject. This shutter arrangement 20 can in principle be arranged at any suitable position in the imaging instrument 1 including the entire light path from each one of the individual light sources over the common light path 150 to a location of the ophthalmic imaging instrument 1 at which the scanning beam B leaves the scope of effective control before eventually reaching a target. Preferably, the shutter arrangement 20 can be positioned in the optical path after the combining optics 15 which facilitates control over all partial and contributing beams at one location by means of one element. Also, preferably, the shutter arrangement 20 can be positioned in the optical path before a first scanning element where the beam of light B follows a stationary path without varying in direction and propagation angle. In this way, the beam can be blocked by shutting the propagating path at a locally fixed point corresponding to the beam cross section without the need for considering any varying directions or angles, which, in turn, can keep the shutter arrangement small in size and low in structural complexity.

Further, in the ophthalmic imaging instrument 1 the controller 140 is configured to operate the shutter arrangement 20 to block the beam of light B when switching between a first imaging modality and a second imaging modality. Specifically, the second imaging modality may employ one or more additional light sources as compared to the first imaging modality. This may imply that the switching from the first imaging modality to the second imaging modality may include activating one or more of the plurality of light sources, which, in turn, may involve power fluctuations especially in a time after activation. For example, a light source may require some time in order to reach a stable power control regime or a state in which the optical power fluctuates only within a permissible band ranging from a nominal minimum optical power to a nominal maximum power. As already mentioned, however, the optical power may play an important role in the context of scanning laser ophthalmoscopy (SLO) as (human) tissue in general and the eye in particular are subject to a more or less strict power budget. In a sense, there is a given maximum power budget that is permissible during an examination in terms of both a maximum instantaneous power as well as a power budget in the form of an integral of the applied optical power over the time of an examination. Therefore, any power fluctuations may exceed a permissible instantaneous optical power applied and/or may add substantial uncertainty as regards the calculation of, and, ultimately, the compliance to a power budget.

Thus, preferably, the beam of light can be blocked by the controller before one or more light sources are activated as part of switching between a first imaging modality and a second imaging modality. However, the beam of light can also be blocked by the controller before one or more light sources are deactivated as part of switching between a first imaging modality and a second imaging modality, as also the powering off of a light source may result in undefined and fluctuating power behaviour, especially also including power spikes as a result of deactivating any power control loops, which, in turn, may involve switched power supplies as a basis for driving a light emitting element in the form of lasers, diodes, and the like. As an example, the beam of light can be blocked at least 0.5 seconds before one or more light sources are activated and/or at least 0.5 seconds before one or more light sources are deactivated.

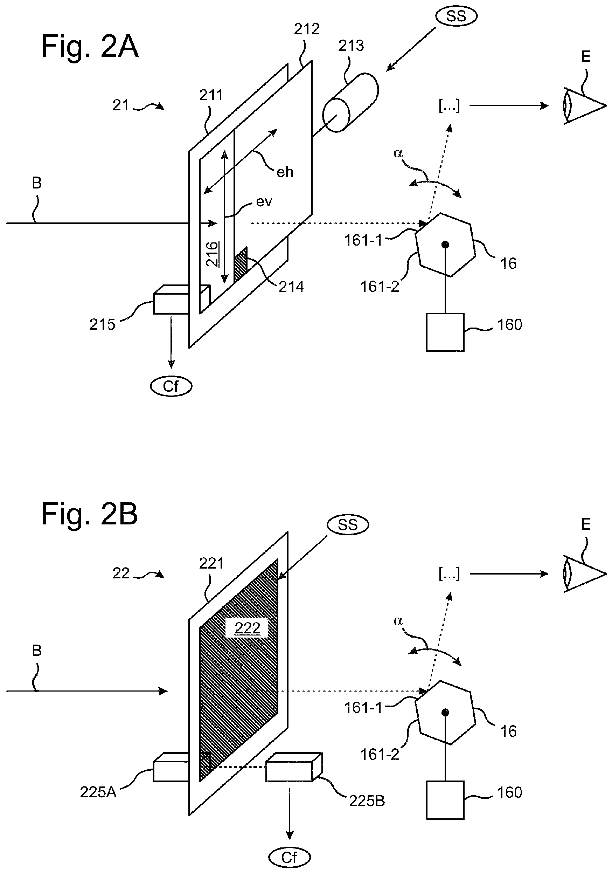

Figure 2A shows a schematic view of an implementation of a shutting arrangement in ophthalmic imaging instruments according to a more specific device embodiment of the present invention. In this embodiment it is first envisaged that the beam of light to be blocked propagates along a constant direction and angle, which may provide advantages in relation to size and complexity of the configuration. Such aspects may be supported by positioning the shutter arrangement at a location before a first scanning element such as a scanning mirror in the form of a scanning polygon mirror or galvo in the optical path. In the present embodiment a mechanical shutter arrangement is shown, which can provide a high reliability as well as further safety mechanisms as a physical shutter can also interact with switches, sensors, light barriers and the like for providing a confirmation feedback as explained below. As shown, the beam of light B is incident from one or more light sources and propagates toward a first scanning element as shown in the example of a scanning polygon mirror 16, which provides for a plurality of reflecting facets 161-1, 161-2, .... A mirror driver 160 is usually arranged to rotate during operation of the body of the polygon scanning mirror 16, such that each facet 161-1, 161-2,... can reflect an incident beam of light at a varying angle α. During the passing of one individual facet, the beam of light is deflected in a corresponding range of a varying angle. After completion of such a deflection cycle, the next facet substantially repeats this process and so do all other facets.

The shown shutting arrangement 21 comprises a frame 211 and a moveable shield 212 that can be positioned relative to the frame 211 by means of a driver 213. Specifically, the shutter arrangement 21 can comprise the movable shield 212, under actuation of the driver 213, can close an aperture 216 of the shutter arrangement to block the beam of light in response to a shutting signal. In the shown example, the aperture 216 is defined by the frame 211. The driver 213 can comprise and implement any suitable mechanical actuating mechanism, such as a magnet, an electromagnet, a spring, piezo elements and the like, that move the shield 212 into the path of the beam of light B in response to a shutting signal SS. Such mechanisms are as such known and can well provide reliable operation even at time scales in the range of milliseconds.

The shield 212 can be an opaque element that can block the light beam, thereby preventing the beam from propagating towards the patient's eye, when the shield 212 is in a beam blocking position. Preferably, the shield 212 comprises a section 214 that is configured to interact with a confirmation sensor 215 so as to provide a confirmation signal Cf. The confirmation sensor 215 may comprise any one of a mechanical switch, a pair of electric contacts, a light barrier in turn comprising a light source and a light detector, a reflecting surface, a magnet and a Hall sensor, an amplifier, a threshold circuit such as a Schmitt trigger and the like. In this way, the confirmation sensor 215 can be configured to provide the confirmation signal Cf in response to detecting an actual position of the shield 212 and, with this, an actual blocking action of the beam by the shield 212 when the shield 212 is in the blocking position. This confirmation signal Cf may be employed by the control 140 in order to confirm blocking of the beam in response to launching the shutting signal SS. For example, the control 140 may be configured to only proceed with changing an imaging modality if the confirmation signal Cf confirms shutting of the propagation path of the beam of light B.

Although this embodiment primarily envisaged that the beam of light to be blocked propagates along a constant direction and angle, a modification can be provided for a beam of light that propagates at a varying direction and angle of incidence, as is, for example, the case for a scanning beam that was already subject to scanning deflection upstream in the optical path. For such a modification, the frame 211 and shield 212 may be configured to have an aperture size having for example a sufficient extensions ev and eh in one or two dimensions so as to accommodate for the applicable angle range in an open, non-blocking state, but to block the beam for all occurring directions and angles in a closed blocking state.

Figure 2B shows a schematic view of an implementation of a shutting arrangement in ophthalmic imaging instruments according to a more specific device embodiment of the present invention. In this embodiment it is first envisaged that the beam of light to be blocked propagates along a constant direction and angle, which may provide advantages in relation to size and complexity of the configuration. Such aspects may be supported by positioning the shutter arrangement at a location before a first scanning element such as a scanning mirror in the form of a scanning polygon mirror or galvo in the optical path. In the present embodiment an optical shutter arrangement is shown, which can provide a high reliability as it may not need to rely on any moving parts. As shown, the beam of light B is incident from one or more light sources and propagates toward a first scanning element as shown like in conjunction with Figure 2A in the example of a scanning polygon mirror 16. The shown shutting arrangement 22 comprises a frame 221 and an optical shield 222 that can be driven by a shutting signal SS so as to block any light. In this embodiment, the optical shield 222 can be being switched between a transparent state and an opaque state by means of the control signal SS being applied to the optical shield 222. Such shields may be implemented by any one of a polarizer and polarization filter, in which, for example, a control signal SS in the form of a voltage may alter polarization properties of a layer of the optical shield 222 so as to respectively set one of the transparent and the opaque state. In one example, the polarization properties of a liquid crystal layer can be changed by means of the control signal SS which in combination with a polarization filter can form an optical shield which can be switched between a transparent state and an opaque state. Other possible implementations may involve Pockels-cells.

Preferably, the shutting arrangement 22 may again include a confirmation sensor in the form of a light barrier having a light emitter 225A and a light detector 225B so as to provide a confirmation signal Cf. In case of the optical blocking mechanism, the confirmation sensor 225 is thus implemented in the form of a light barrier for which the light shares the aperture with the beam of light B, implying that if the light barrier indicates that no light passes, then the beam of light B is blocked.

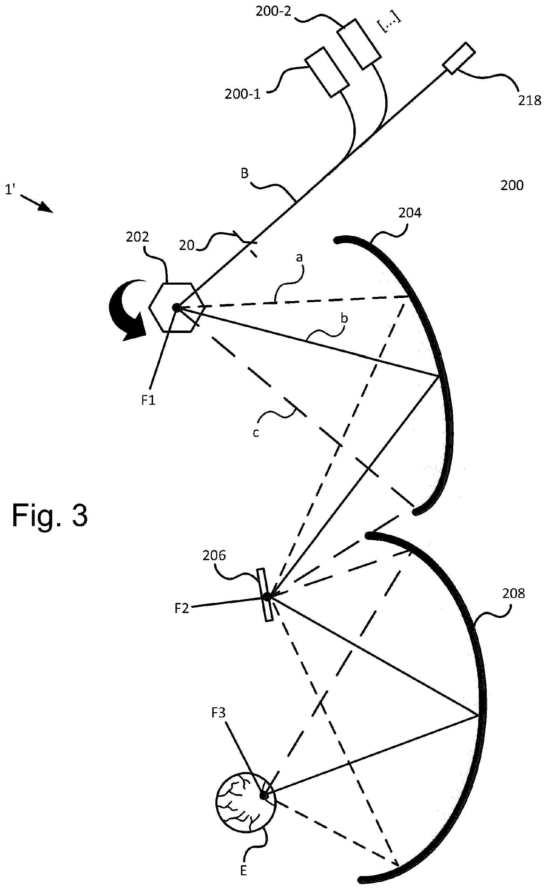

Figure 3 shows a schematic view of an ophthalmic imaging instrument for providing more than one imaging modality according to a device embodiment of the present invention. The ophthalmic imaging instrument 1' is described in the context of an imaging modality in which a beam of light B is generated by one or more light source(s) 200-1, 200-2,... and guided toward a patient's eye E, reflected at tissue of the same and guided back to a detector 218. The instrument 1' thus includes light sources that emit a beam of light in the form of a scanning beam by means of a plurality of scan relay elements. The scan relay elements include a first scanning element in the form of a polygon scanning mirror 202, a second scanning element 206, and optical elements that are positioned and configured to direct the scanning beam B. In some embodiments, the optical elements may include the optical element 204 which may be a scan compensation element such as an ellipsoidal mirror (and referred to as a slit mirror), and a second optical element 208 which may be a scan transfer element which may also be an ellipsoidal mirror.

The optical elements 204 and 208 are positioned and configured to direct the scanning beam B. The second scanning element 206 may be or include an oscillating plane scanning mirror or a planar scanning mirror coupled to a galvanometer motor. The optical element 204 may be a curved mirror such as an ellipsoidal mirror. The second optical element 208 may be an aspherical mirror. It is to be appreciated that the first and second optical elements may have an alternative form. The scanning elements 202 and 206 may be referred to as a scanning device or a plurality of individual scanning devices. It is to be understood that the shown functionalities are just an example of a configuration that can be used with the embodiments described herein. In some embodiments, one or more of the scanning elements may include one or more of: an oscillating plane mirror, a galvanometer mirror, a MEMS mirror, a rotating mirror, prism or polygon scanner, and/or a resonant mirror, for example. It should be appreciated that other suitable scanning elements may be used, such as line scanning produced with a laser line source, or equivalent. Line scanning may be used as an effective alternative to point scanning. A line source may produce a line illumination on the retina which is scanned orthogonally by a slow scanner. The line illumination may be detected by a linear pixel array, and a 2D image may be built up by rotating the slow scanner.

In some embodiments, each of the first scanning element 202 or the second scanning element 206 may be a single element or an arrangement of two or more elements as suitable to provide a scan at a respective focal point F1 or F2, as shown, at which the scanning element is disposed. The focal points F1 and F2 are the foci of the optical element 204, and the focal points F2 and F3 are the foci of the optical element 208. The first scanning element 202 is positioned at focal point F1, the second scanning element 206 is positioned at focal point F2, and the eye E is positioned at focal point F3 (also referred to as a virtual scanning point). The resulting scan may be a 2D scan or scan pattern of the scanning beam 201 as the light sweeps through the virtual scanning point (e.g., through F3) inside the eye E.

In some embodiments, the first scanning element 202 provides either a vertical, horizontal, or patterned scan which is incident on the optical element 204, to a point on the second scanning element 206 via the optical element 204. The scans may be one-dimensional (1D) or two-dimensional (2D) light scans, for example. The axes of the first scanning element 202 and the second scanning element 206 may be arranged to create a 2D light scan, such as in the form of a raster scan pattern of the scanning beam 201. The alignment of the first and second scanning elements 202, 206 may be orthogonal, substantially orthogonal, or arranged to generate an arbitrary scan geometry about the optical elements 204 and 208.

In some embodiments, the second scanning element 206 provides a plurality of scans, for example 1D or 2D light scans, which may comprise horizontal scans, vertical scans, or arbitrary patterns of the scanning beam 201. The scans provided by the first scanning element 202 and the scans provided by the second scanning element 206 are different from each other, for example with respect to the orientation of the scans. In some examples, one of the scanning elements may provide vertical scans of the retina, and the other scanning element may provide horizontal scans of the retina. The scanning beam 201 is directed towards a patient's eye E via the scanning elements 202 and 206, and the optical elements 204 and 208, such that an ultra-wide field scan angle is achieved at the pupil plane of the eye E.

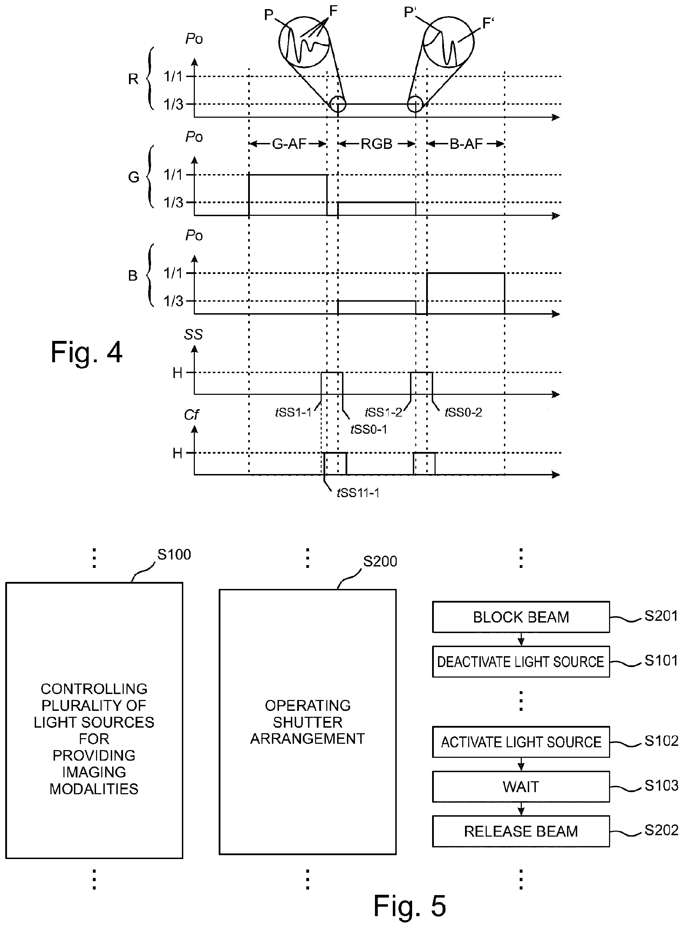

In some examples, the plurality of line scans may be generated by scanning the retina along a first direction using the first scanning element 202, and positions of the plurality of line scans are varied along a second direction using the second scanning element 206, where the second direction is orthogonal to the first direction. As illustrated in Figure 4, the path of the scanning beam B is shown in a 1D scan produced by one oscillation or rotation (shown by the curved arrow) of the first scanning element 202. Path "a" is an example of the scanning beam B reflected from the polygon scanning mirror at one orientation of a reflecting facet during rotation, whereas paths "b" and "c" are examples of the scanning beam B reflected at other orientations of the facet during rotation.

The components of the ophthalmic imaging instrument 1' may be arranged such that the rotational axis of the first scanning element 202 is substantially parallel to a line joining the two foci of the optical element 208 (i.e., F2 and F3) such that the scanning beam B is scanned across the secondary axis of the optical element 204. Furthermore, the first scanning element 202 may produce a 1D or 2D scan which is incident on the optical element 204. The optical element 204 may also therefore produce a 1D or 2D scan. The components of the ophthalmic imaging instrument 1' may be arranged such that the line joining the two foci of the optical element 208 (i.e., F2 and F3) lies substantially on a plane defined by the scan (e.g., 1D vertical scan) produced by the optical element 204. The first and second scanning elements 202 and 206 may thus together create a light scan, for example a 2D scan, in the form of a raster scan pattern from a single point in space at or near the focal point F3 at or in the eye E of the patient. The first and second scanning elements may have operating parameters which include the amplitude of the oscillation and the rotational offset of the oscillation. The operating parameters also include the velocity of oscillation. Both of these operating parameters may be selected to control the direction and pattern of the light scan from the apparent point source. In some examples, the first and second scanning elements may be housed in a rotation mount (not shown) that can adjust the centering (or eccentricity) of the scanning beam B on the retina of the eye E, which provides the ability to "move" the imaging field across the retina.

In the present embodiment, the ophthalmic imaging instrument 1' comprises a shutting arrangement 20 which is positioned to block the beam of light B incident from the one or more light sources before it propagates further in the optical path, specifically before propagating further toward the first scan relay element in the form of the scanning polygon mirror 202. At this point, the beam of light B propagates at a substantially constant direction and angle so that the shutting arrangement 20 can advantageously block the beam of light B although it is employed to ultimately form a scanning beam for covering some kind of finite imaging area. As regards any further implementation details in the context of the present embodiment it is referred to the disclosure rendered in conjunction with Figures 2A & 2B.

Figure 4 shows schematic views of optical powers for individual beams of light under different imaging modalities and other related driving signals according to an embodiment of the present invention. Specifically, there are shown different imaging modalities in terms of respective target optical powers for three light sources R, G and B, wherein such light sources can comprise respective lasers that emit light in a red colour (R), a green colour (G) and in a blue colour (B). The mentioned lasers can be an implementation of the plurality of light sources 11-1, 11-2,... of Figure 1 or 200-1, 200-2,... of Figure 3. In a first imaging modality only one light source is active and is to use the full optical power budget by providing an optical power Po being denoted by 1/1. This imaging modality can be a green auto-fluorescence (G-AF) imaging modality, in which only green light is used. In such a modality, the gain can be set so that some desired output is produced, e.g. an output voltage of 1V, when the optical power of the green light is at a given target power, e.g. 2 mW. In a second imaging modality all three light sources R, G and B are active and each light source can only use a fraction of the optical power budget. For example, each light source can be operated to provide only a third of the optical power 1/1, which is denoted by 1/3. This imaging modality can be a full colour or RGB imaging modality, in which white light is used.

Possible variations the actual optical power in connection with activating and deactivating a light source are shown and explained with the example of the operation of the red laser R between the imaging modality G-AF and RGB, and, respectively, between the imaging modality RGB and B-AF (see insets). In that example, the switching from the first imaging modality G-AF to the second imaging modality RGB involves activating (turning on) a light source in the form of the red laser R. This may, in turn, involve power fluctuations F and/or power spikes S as shown in the left inset. In order to avoid any disadvantageous effects in connection with these, a shutting signal SS may activate a shutting arrangement as this has been explained in conjunction with the present disclosure. Specifically, the shutting signal SS may control such a shutting arrangement to block the beam of light propagating toward an eye of a subject up to a time tSS0-1 when the power fluctuations F can be safely assumed to have disappeared or any further power spikes S are - at least - unlikely to occur. The same or at least similar considerations apply for the shutting signal SS at a time tSS0-2 in connection with a switch from the RGB imaging modality to the imaging modality B-AF.

However, power fluctuations and/or power spikes may not only occur in connection with activating a light source but also when deactivating (turning off) a light source. In order to consider such effects, the shutting signal SS may control the shutting arrangement to block the beam of light propagating toward an eye of a subject from a time tSS1-1 which may even precede a point in time when a light source is deactivated. In this way, any power fluctuations F' and/or power spikes S' can be safely blocked. The same or at least similar considerations apply for the shutting signal SS at a time tSS1-2 in connection with a switch from the RGB imaging modality to the imaging modality B-AF. Therefore, the controller can be configured to operate the shutter arrangement to block the beam of light when switching between a first imaging modality and a second imaging modality by specifically blocking the beam of light before a light source is activated and/or before a light source is de-activated.

As a further option, there may be considered a confirmation signal Cf which may originate from a confirmation sensor as explained elsewhere in the present disclosure. Specifically, such a confirmation signal may represent a confirmation that the beam of light propagating toward an eye of a subject is effectively and actually blocked. For example, such a signal may be received with some delay at a time tSS11-1 in reaction to launching the shutting signal at time tSS1-1, as the latter controls the shutter arrangement to close and confirmation sensor (with said delay) registers and confirms the closure of the light path. Specifically, the control may first control the shutting arrangement to block the beam of light propagating toward the eye of a subject by launching a corresponding shutting signal SS (e. g. setting the signal to a relatively high logic level H), the wait for the confirmation signal Conf to react (e. g. by transitioning to a relatively high logic level H, and only then to proceed with activating and/or deactivating any light source in connection with switching from a first imaging modality to a second imaging modality.

Figure 5 shows a flowchart of a general method embodiment of the present invention. Specifically, this embodiment relates to a method of operating an ophthalmic imaging instrument. For example, there can be provided a method of operating an ophthalmic imaging instrument 1 that comprises a plurality of light sources 11-1,... for providing a beam of light and an optical path comprising one or more scanning elements 16, 18 and one or more guiding elements 17, 19 configured to project the beam of light to an eye of the subject, and a shutter arrangement 20 configured to block the beam of light so that it does not reach the eye of the subject. The method comprises a step S100 of controlling the light sources to generate a beam of light and to provide a plurality of imaging modalities, wherein a corresponding contribution from the light sources to a power of light projected toward an eye of a subject varies for each one of said imaging modalities. The method comprises further a step S200 of operating the shutter arrangement to block the beam of light when switching between a first imaging modality and a second imaging modality. It is noted that these steps S100 and S200 can be performed sequentially, repeatedly and/or concurrently depending on the actual implementation.

In some embodiments, the shutter arrangement is operated to block the beam of light in a step S201 before a step S101 of deactivating a light source in connection with ending a first imaging modality. Further, the shutter arrangement can be operated to block the beam of light at least until a delay after activating a light source in connection with starting a second imaging modality. Specifically, the respective order may consider a first step S102 of activating at least one light source, a second step S103 of waiting a predetermined time (e.g. a delay time for which it can be assumed that any fluctuations or spikes in the actual optical power have ceased, and then a step S202 of releasing the beam of light by operating the shutter arrangement so as to no longer block the beam of light.

The forgoing has presented embodiments and details thereof as part of the present invention which can provide one or more advantages by improving power control mechanisms in ophthalmic imaging instruments that allow for the flexibility of providing a plurality of imaging modalities but maintain safety while not requiring an increase in system and/or implementation. While various embodiments of the present disclosure have been described above, it should be understood that they have been presented by way of example, not limitation. It will be apparent to persons skilled in the relevant art(s) that various changes in form and detail can be made therein. Thus, the above-described exemplary embodiments are not limiting.

## Claims

1. An ophthalmic imaging instrument (1) comprising:
a plurality of light sources (11-1, 11-2, 11-3) for providing a beam of light (B);
a controller (140) arranged to control the light sources (11-1, 11-2, 11-3) to generate the beam of light (B) and to provide a plurality of imaging modalities, wherein a corresponding contribution from the light sources (11-1, 11-2, 11-3) to a power of light projected toward an eye (E) of a subject varies for each one of said imaging modalities;
an optical path (150) comprising one or more scanning elements (16, 18) and one or more guiding elements (17, 19) configured to project the beam of light (B) to an eye (E) of the subject; and
a shutter arrangement (20) configured to block the beam of light (B) so that it does not reach the eye (E) of the subject,
wherein the controller (140) is configured to operate the shutter arrangement (20) to block the beam of light (B) when switching between a first imaging modality and a second imaging modality.

2. The ophthalmic imaging instrument (1) according to claim 1, further comprising combining optics (15) configured to combine beams of light (B-1a, B-2a, B-3a) originating from the individual ones of the plurality of light sources (11-1, 11-2, 11-3) for providing the beam of light (B).

3. The ophthalmic imaging instrument (1) according to claim 2, wherein the shutter (20) arrangement is positioned in the optical path (150) after the combining optics (15).

4. The ophthalmic imaging instrument (1) according to any one of claims 1 to 3, wherein the shutter arrangement (20) is positioned in the optical path (150) before a first scanning element (16).

5. The ophthalmic imaging instrument (1) according to any one of claims 1 to 4, wherein the controller (140) is arranged to control a first number of light sources to provide a first imaging modality, and to control a second number, being different from said first number, of light sources to provide a second imaging modality.

6. The ophthalmic imaging instrument (1) according to any one of claims 1 to 5, wherein the controller (140) is arranged to activate at least one of the light sources (11-1, 11-2, 11-3) or to deactivate at least one of the light sources (11-1, 11-2, 11-3) when switching from the first imaging modality to the second imaging modality.

7. The ophthalmic imaging instrument (1) according to claim 6, wherein the controller (140) is configured to operate the shutter arrangement (20) to block the beam of light (B) before at least one light source is activated when switching between the first imaging modality and the second imaging modality.

8. The ophthalmic imaging instrument (1) according to claim 6 or 7, wherein the controller (140) is configured to operate the shutter arrangement (20) to block the beam of light (B) after at least one light source is deactivated when switching between the first imaging modality and the second imaging modality.

9. The ophthalmic imaging instrument (1) according to any one of claims 1 to 8, wherein the shutter arrangement (21) comprises a movable shield (212) and a driver (213) configured to close an aperture (216) of the shutter arrangement (20) to block the beam of light (B) in response to a shutting signal (SS).

10. The ophthalmic imaging instrument (1) according to any one of claims 1 to 8, wherein the shutter arrangement (22) comprises an optical shutter (222) configured to block the beam of light (B) in response to a shutting signal (SS).

11. The ophthalmic imaging instrument (1) according to any one of claims 1 to 10, further comprising a confirmation detector (215, 225A/B) configured to provide a confirmation signal (Cf) in response to the beam of light (B) being blocked by the shutter arrangement (20, 21, 22).

12. The ophthalmic imaging instrument (1) according to claim 11, wherein the controller (140) is configured to operate the shutter arrangement (20) to block the beam of light (B), receive the confirmation signal (Cf) and to control the light sources (11-1, 11-2, 11-3) so as switch between the first imaging modality and the second imaging modality in response to receiving said confirmation signal (Cf).

13. A method of operating an ophthalmic imaging instrument (1) comprising a plurality of light sources (11-1, 11-2, 11-3) for providing a beam of light and an optical path (150) comprising one or more scanning elements (16, 18) and one or more guiding elements (17, 18) configured to project the beam of light (B) to an eye (E) of the subject, and a shutter arrangement (20) configured to block the beam of light (B) so that it does not reach the eye (E) of the subject, the method comprising the steps of:
- controlling (S100) the light sources (11-1, 11-2, 11-3) to generate a beam of light (B) and to provide a plurality of imaging modalities, wherein a corresponding contribution from the light sources (11-1, 11-2, 11-3) to a power of light projected toward an eye (E) of a subject varies for each one of said imaging modalities; and
- operating (S200) the shutter arrangement to block the beam of light (B) when switching between a first imaging modality and a second imaging modality.

14. The method of operating an ophthalmic imaging instrument (1) according to claim 13, further comprising a step of controlling a first number of light sources to provide a first imaging modality, a step of controlling a second number, being different from said first number, of light sources to provide a second imaging modality, and a step of activating (S102) at least one of the light sources (11-1, 11-2, 11-3) or deactivating (S101) at least one of the light sources (11-1, 11-2, 11-3) when switching from the first imaging modality to the second imaging modality.

15. The method of operating an ophthalmic imaging instrument (1) according to claim 14, further comprising a step of operating a shutter arrangement to block the beam of light before at least one light source is activated when switching between the first imaging modality and the second imaging modality.
